(19) **Europäisches Patentamt**

**European Patent Office**

**Office européen des brevets**

(11) Publication number: **0 138 915**
**B1**

(12) **EUROPEAN PATENT SPECIFICATION**

(45) Date of publication of patent specification: **07.06.89**

(21) Application number: **84901298.4**

(22) Date of filing: **09.03.84**

(86) International application number:
**PCT/US84/00352**

(87) International publication number:
**WO 84/03620 27.09.84 Gazette 84/23**

(51) Int. Cl.⁴: **A 61 F 5/43**

(54) **CONTRACEPTIVE HOOD.**

(30) Priority: **16.03.83 US 475852**
**10.02.84 US 577602**

(43) Date of publication of application:
**02.05.85 Bulletin 85/18**

(45) Publication of the grant of the patent:
**07.06.89 Bulletin 89/23**

(84) Designated Contracting States:
**FR GB NL SE**

(56) References cited:
**GB-A-2 106 784**
**US-A-2 389 831**
**US-A-3 648 700**
**US-A-3 677 225**
**US-A-3 788 324**
**US-A-3 951 141**
**US-A-4 187 851**
**US-A-4 284 079**
**US-A-4 320 752**

(73) Proprietor: **Mentor Corporation (a corporation of the State of Minnesota)**
**1499 West River Road**
**Minneapolis, Minnesota 55411 (US)**

(72) Inventor: **CONWAY, Anthony James**
**Rural Route 1**
**Chatfield, MN 55923 (US)**
Inventor: **CONWAY, Peter Ralph**
**107 Twiford Street**
**Chatfield, MN 55923 (US)**
Inventor: **CONWAY, Philip Joseph**
**513 N.E. 10th Avenue**
**Stewartville, MN 55976 (US)**

(74) Representative: **Sheard, Andrew Gregory et al**
**Kilburn & Strode 30, John Street**
**London WC1N 2DD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### BACKGROUND OF THE INVENTION
### 1. Field of the Invention.

The present invention relates to male contraceptive hoods which are adhesively secured to the penis, the hood being initially rolled up and the interior of the hood being free of adhesive until it is unrolled.

### 2. Description of the Prior Art.

The conventional contraceptive condom is designed to cover not only the penile head but the entire penile shaft. It is used usually initially in a rolled up condition and is unrolled to extend for substantially the full length of the penile shaft. It is retained in position largely by friction between the inner wall of the condom and the penis. An objection to condoms of this type is that they tend to slip off the penis after ejaculation has occurred and the penis tends to assume a flaccid condition. This may result in semen accidentally entering the vaginal passage.

Recognizing the drawback of the conventional condom, various attempts have been made to provide a contraceptive hood which is adhesively secured to the penis. The difficulty with such a device is that it will not remain in place unless adhesively secured to the head of the penis. The early United States Woodruff patent, Patent 822,092, for example, shows a short contraceptive hood in which there is adhesive applied to the interior. It is obviously very difficult to apply such a contraceptive hood when adhesive is already in place. Furthermore, the arrangement of the Woodruff patent has the drawback that it provides a long extending nipple which would interfere with the sexual act. The United States Patents to Kopelowicz, Patent 3,951,141; Wayne, Patent 2,448,938; and Czirely, Patent 3,677,225 all show arrangements in which there is an adhesive over a portion of the interior of a short contraceptive hood with a release strip of some kind which is stripped off in connection with applying the contraceptive hood to the penis. The arrangements of these patents all have the drawback, however, in that a separate release strip is necessary and that the adhesive is not always located where it is most effective. The Ormo patent 2,839,060 shows an arrangement in which adhesive is applied on the inner surface of an inner sheath. There is also a ridge that acts as a sealing device. The device can be relatively complicated to apply partly due to the continual presence of the adhesive on the inner surface. The Warner Patent 3,648,700 shows a contraceptive device in which there are bands which are used for overlapping encirclement of the penis behind the tip, these overlapping bands being coated on the inside with adhesive. Such an arrangement provides an adhesive over only a very limited area of the contraceptive hood and further provides a very irregular surface due to the space between the strap and the main portion of the hood and also due to a thickened portion resulting from the overlapping portions of the straps. The Welsh Patent 2,389,831 shows a protective covering for a member of the body, such as finger or toe. Initially, there is adhesive on an outer surface. This is covered by a protective tape which must be removed. In order to bring the adhesive into the inner surface, the entire unit is turned inside out. This would be very difficult to manipulate as a contraceptive device.

### SUMMARY OF THE INVENTION

The present invention is concerned with a contraceptive hood in which there is no adhesive on the interior of the hood until the hood has been unrolled on the head of the penis. Therefore according to the present invention there is provided a contraceptive hood to be adhesively applied to a penis, said contraceptive hood including:

a flexible cylindrical member (12) having a closed end and an open end;

said cylindrical member (12) being rolled outwardly upon itself from said open end to form consecutively larger rolls (13);

and an adhesive layer (15) and an adhesive release layer (14) disposed between the rolls (13) and the adhesive release layer (14) disposed between said adhesive layer (15) and the outer surface of said cylindrical member (12) in non-adhering contact with the adhesive layer (15) so that upon said member (12) being unrolled onto the head of a penis (20), the adhesive layer (15) adheres to the inner surface of said cylindrical member (12) to cause the cylindrical member (12) to adhere to the penis(20). Facilitation of the transfer of adhesive from the outer surface of the cylindrical member to the inner surface, is achieved by the release layer interposed between the adhesive layer and the outer surface of the cylindrical member.

In one form of the invention, the cylindrical member is in the form of a thin member and an adhesive layer and a release layer are applied directly to the outer wall of this thin cylindrical member. In a preferred form, the cylindrical member comprises an inner thin cylindrical portion and an outer relatively thick cylindrical portion, the outer relatively thick cylindrical portion bearing the adhesive layer and the release layer so that the adhesive is transferred to the inner surface of the thin cylindrical member as the unit is unrolled.

The hood may be relatively short so as to cover only the head of the penis or may be sufficiently long to cover the entire penile shaft.

In the modification in which there is both an inner thin portion and an outer thick portion, the outer thick portion is removed after the contraceptive hood is in place. This can be readily accomplished since the adhesive has been transferred to the inner surface of the inner thin portion. To facilitate such removal, the outer thick portion may be provided with a projecting portion which may be grasped to pull it loose from the inner thin portion. To facilitate withdrawal of the unit, the outer thick portion is provided with a small aperture to permit air to enter between the inner thin member and the outer thick member as the latter is withdrawn so as to prevent a vacuum being formed in the outer portion is withdrawn.

The adhesive layer is preferably applied over that portion of the length of the hood adjacent the open end which is engaged by the inner surface of the cylindrical member when the cylindrical member is in rolled up condition.

Various other objects and features of the invention will be apparent from a consideration of the accompanying specification, claims and drawing.

BRIEF DESCRIPTION OF THE DRAWINGS

Figure 1 is a perspective view of one form of our contraceptive hood prior to the same being applied to a penis;

Figure 2 is a sectional view of the contraceptive hood, the section being taken along the line 2--2 of Figure 1;

Figure 3 is a sectional view on a higher enlarged scale of a portion of the hood showing how the adhesive is transferred from the outer surface of the cylindrical member to the inner surface;

Figure 4 is a view showing the contraceptive hood of Figures 1 and 2 in place on the penis;

Figure 5 is a perspective view of a modified and preferred form of our invention;

Figure 6 is a sectional view taken along the line 6--6 of Figure 5;

Figure 7 is a view of the thin inner member of the contraceptive hood of Figures 5 and 6 placed over a mandrel;

Figure 8 is a view showing the thick outer portion of the cylindrical member which has been applied to the thin inner portion while still on the mandrel;

Figure 9 is similar to Figure 8 showing a release layer and an adhesive layer which have been applied to the outer surface of the thicker outer portion;

Figure 10 is a view on a highly enlarged scale of a portion of the contraceptive hood of Figures 5 and 6 which is shown as being rolled up to show the various layers of the hood;

Figure 11 is a view similar to Figure 10 but showing the hood being unrolled to show the transfer of adhesive from the outer surface of the outer member to the inner surface of the inner member;

Figure 12 is a view showing the contraceptive hood of Figures 5 and 6 being applied to the tip of a penis, the device still being partially rolled up;

Figure 13 is a view similar to Figure 12 but with the contraceptive hood unrolled on the tip of a penis.

Figure 14 is a view showing the hood after the outer cylindrical member has been removed;

Figure 15 is a perspective view of a modification of the outer member as far as means for removing the outer cylindrical member is concerned;

Figure 16 is a view of a further modification of our invention in which there is a two-part hood as in Figures 5-14, but in which the condom hood covers substantially the entire penile shaft, the device being shown in Figure 16 as being initially applied to the penis;

Figure 17 is a view of the same hood with the outer member completely unrolled and the inner member unrolled to the base of the penis;

Figure 18 is a view in which the outer member has been pulled away from the inner member; and

Figure 19 is a sectional view on a large scale showing a portion of the contraceptive hood of Figures 16-17 being rolled up and showing the various layers of the hood.

DETAILED DESCRIPTION OF THE PREFERRED EMBODIMENTS

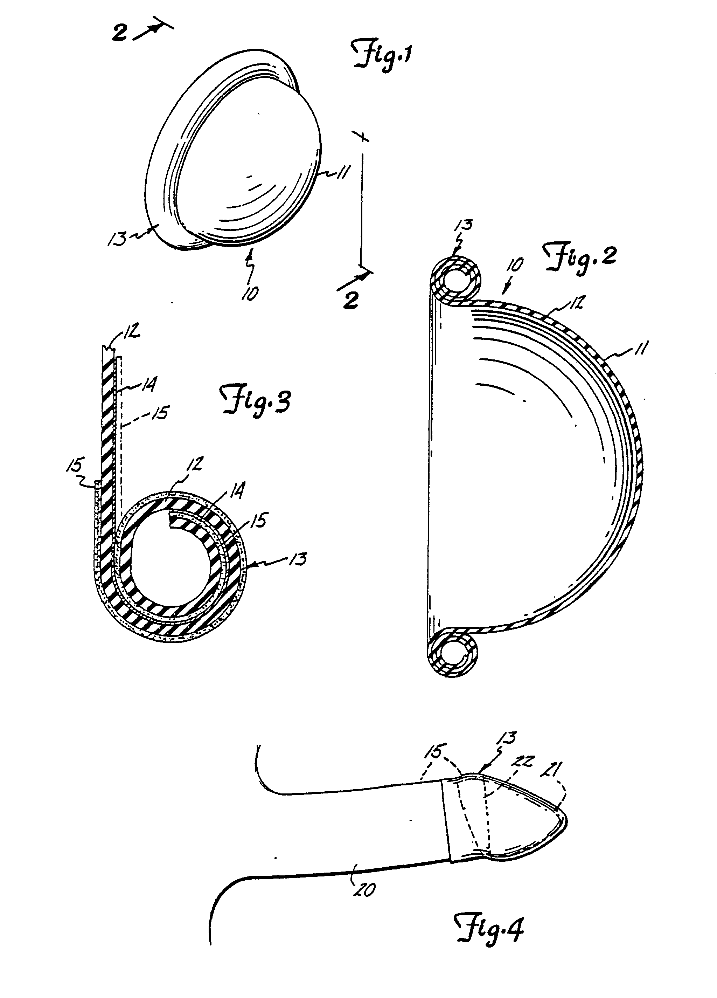

Referring to Figure 1, the contraceptive hood, shown in perspective in that figure is designated in its entirety by the reference numeral 10. A hood is formed of a sheath 12 of very thin resilient material which may be latex, for example, the material having a thickness of between 0.005 and 0.01cm (.002 and .004 inches). The hood has a dome portion 11 which is designed to go over the tip of the penis. In the form shown in Figures 1 and 2, the hood is rolled up to form a roll 13. The internal circumference of the roll 13 is such that the hood 10 can be readily fitted and placed over the tip of a penis.

As previously explained, the contraceptive hood of the present invention has a releasable layer of pressure sensitive adhesive which initially is on the outside surface of a portion of the hood but which is transferred to the inner surface upon the hood being unrolled onto the end of the penis. This action is shown in Figure 3. This figure shows on a highly enlarged scale a portion of the sheath 12 after it has been unrolled. In comparing Figures 2 and 3, the left-hand surface of sheath 12 is the inner surface and the right-hand side is the outer surface. Initially, there is a release layer 14 on this outer surface and an adhesive layer 15. The release agent may, for example, be any conventional silicone release agent. This is shown in the rolled up portion of the sheath 12. It will also be noted upon observing the rolled up portion that the adhesive layer 15 is adjacent the inner wall of turns of the sheath 12. Thus, the adhesive layer tends to cling to the inner surface of sheath 12 being released from the outer surface by reason of the release layer 14. It will also be noted from Figure 3 that the layer of adhesive 15 shown on the inner surface of sheath 12 does not extend to the closed end of it, which end, of course, is the dome portion 11. There is no need for adhesive at the tip. Furthermore, since the layer of adhesive 15 is only applied to the sheath 12 where it has overlapped with an outer surface in the coil 13, it will be obvious that there is a substantial terminal portion of sheath 12 which does not have adhesive on it. All of the portion of sheath 12, however, that extends farthest from the tip of the penis is coated. This will be clear from Figure 4.

Referring specifically to Figure 4, it will be noted that the hood has been unrolled onto a penis 20.

In doing so, the tip 21 of the penis is initially placed within the dome 11 and the roll 13 is unrolled. As this unrolling takes place, the adhesive on the outer surface of the wall 12 is transferred to the inner surface. This action is illustrated in Figure 4 where it will be noted that the adhesive extends over a band extending from the open end of the hood 10 to a portion on the head of the penis, defined by a dotted line 22. In most cases, this adhesive will overlap the head. The major portion of the hood extending over the tip 21 of the penis is free of adhesive.

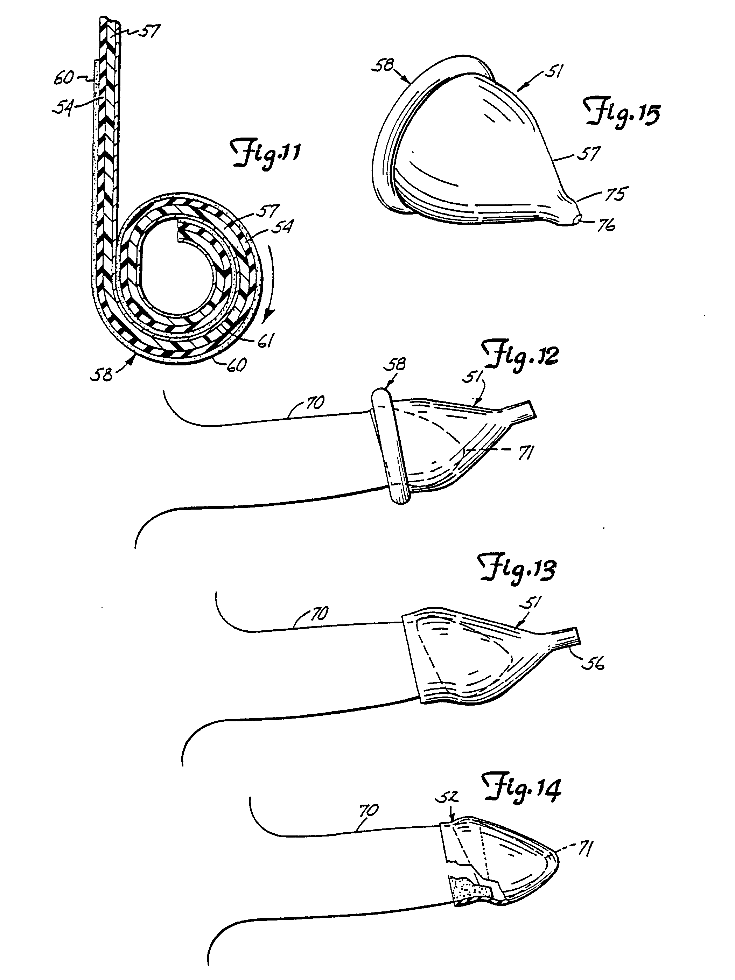

The arrangement of Figure 12 leaves most of the penile shaft of penis 20 bare so as to increase the sensation. At the same time, the hood is held firmly in position regardless of whether the penis is erect or flaccid. There is no preparation necessary since the adhesive is transferred to the inner surface of the hood simply by the normal act of unrolling of the condom hood which necessarily takes place in applying the same to the penis.

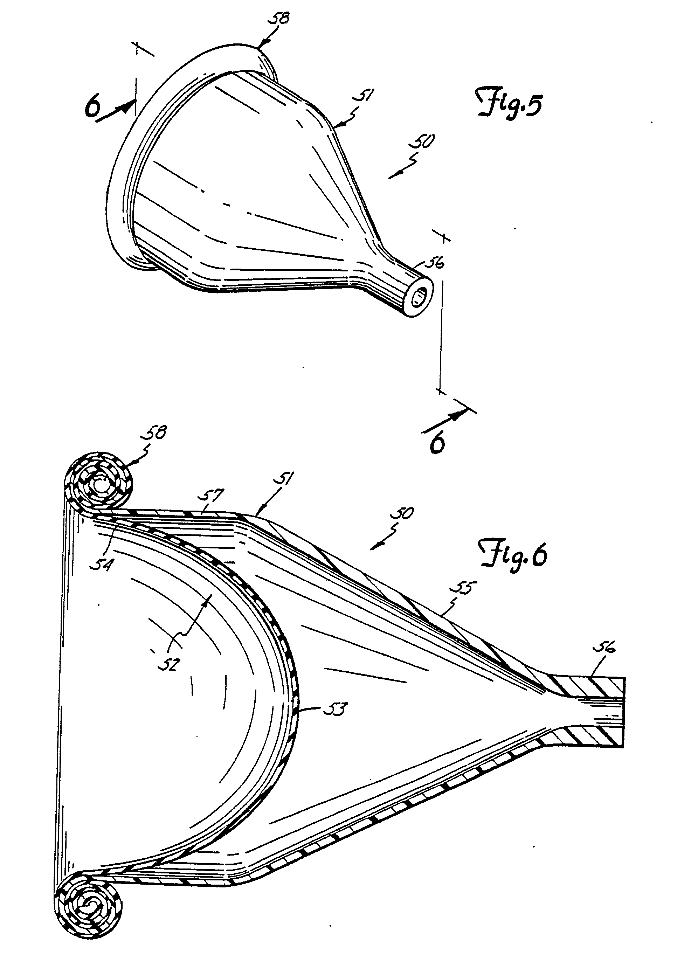

MODIFICATION OF FIGURES 5-15

The contraceptive hood of Figures 1 through 4 is compact and uses a minimum amount of material. It has a drawback, however, that due to the very thin nature of the wall 12 of the contraceptive hood, the roll 13 while shown in highly exaggerated form in Figures 1 through 4 is actually relatively small. It thus becomes somewhat difficult to unroll the roll 13. Moreover, in the arrangement of Figures 1 through 4, the release agent is left on the outside of the hood. This increases the thickness of the hood and thus interferes with the natural sensation. A further problem is that the release agent has a tendency to leave the hood, particularly with the movement accompanying intercourse so that some of the release agent may be left behind in the vagina. In the arrangements of Figures 5 through 19, we have provided alternative and preferred forms in which there is a cylindrical condom hood having an inner portion which remains on the penis and an outer portion which is used merely to assist in applying the hood but which is removed after the condom hood is in place, taking the release agent with it.

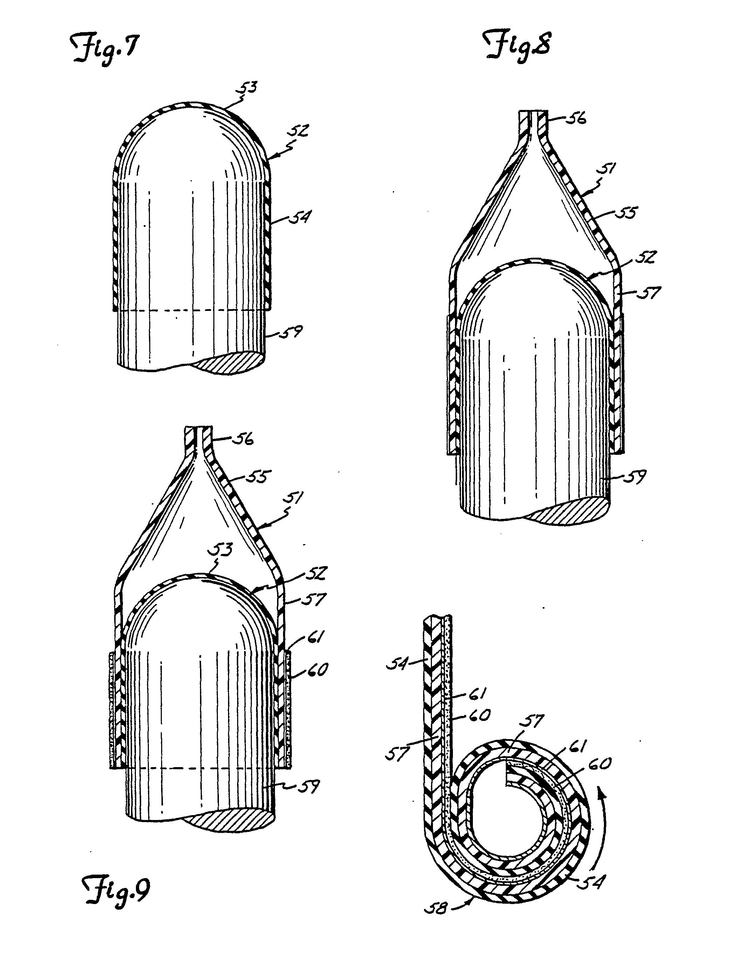

Referring specifically to Figures 5 and 6, the two-piece hood is generally designated by the reference numeral 50. There is an outer portion 51 and an inner portion 52 which is very similar in construction to the condom hood 10 of the species of Figures 1 through 4. In other words, it is made of very thin resilient material having a thickness of between .005 and 0.01cm (.002 and .004 inches). It has a bowl portion 53 and a major sheath portion 54.

The outer portion 51 has a conical portion 55 which terminates in a projecting tubular portion 56. This projecting portion 56, as will be pointed out later, can take various forms so long as it provides a means for grasping the outer member 51 when it is desired to remove it, as will be explained later.

The outer member 51 is made of relatively thick material which may be from 0.038 to 0.064cm (.015 to .025 inches) in thickness and has a body portion 57 which is rolled with the sheath portion 54 to form a roll 58.

As will be explained in more detail later, the outer portion of the cylindrical member 51 is coated with a release layer and an adhesive so that adhesive is transferred in the roll 58 to the inner surface of member 52. After the condom hood is unrolled, the outer member 51 can be pulled loose by grasping the projection 56. It can then be discarded, leaving only the inner member 52 on the penis. The release agent remains on the outer member 51.

Figures 7, 8 and 9 are directed to a method of forming the two part condom hood of Figures 5 and 6.

Referring first to Figure 7, it will be noted that the inner member 52 has been placed upon a mandrel 59 which has a curved upper end corresponding to the curvature of the dome shape portion 53 of the inner member 52.

Referring now to Figure 8, the outer member 51 of the hood has been placed over the inner member 52, the lower ends of both the outer portion 51 and the inner portion 52 terminating at the same point on the mandrel 59.

In Figure 9, there has been applied to the outer surface of the body portion 57 of the outer member 51 an adhesive release layer 61, which may be of the same material as release layer 14 of Figure 3, and a layer of pressure sensitive adhesive 60.

The unit is now rolled up by rolling up the various layers together starting at the lower end. A portion of the sheath in a highly enlarged scale is shown in Figure 10 with the layers in the position they assume as the sheath is rolled up. It will be noted that the release layer 21 and adhesive layer 60 are still on the body portion 57 of the outer member 51. The various layers are rolled together to form a roll 58 and when the rolling is completed, the device will appear as shown in Figures 5 and 6.

Figure 11 is a view similar to Figure 10 but showing the elements as the sheath is being unrolled in its application to a penis. It will be noted that the adhesive layer 60 is now on the inner surface of the sheath 54 of the inner member 52. This has taken place because of the fact that the layer 54 becomes adjacent the inner surface of sheath 57 when the device is in the rolled up position of Figures 10 and 11. Because of the release layer 61, the adhesive adheres to the inner surface of the sheath 54 rather than to the outer surface of the sheath 57 of the outer member 51. Thus, just as in the modification of Figures 1 through 3, the adhesive is transferred from the outer surface to the inner surface.

In Figures 12 through 14, we have shown the method by which the condom hood of Figures 5 and 6 is placed on the penis. As shown in Figure 12, the inner and outer sheath, still rolled together in a roll 58, are placed over the tip 71 of the penis 70.

In Figure 13, we have shown the condom hood as having been unrolled. It will be noted that as in the arrangement of Figures 1 through 3, the unrolled sheath extends over a short portion of the penile shaft beyond the shaft. Because of the fact that the adhesive has been transferred from the outer surface of member 51 to the inner surface of the member 52, it is possible to grasp the projecting portion 56 of the outer member 51 and pull it loose. It can now be pulled off and discarded. Since the release agent has remained on the outer surface of the outer member, it is withdrawn along with the outer member. After the outer member 51 has been pulled off, the device assumes the appearance of Figure 14 which is not greatly unlike that of the appearance of Figure 4. In other words, the adhesive 60 extends over a band beginning at the open end of the inner hood member 52 to a point overlapping the head of the penis.

In Figure 15, I have shown a slight modification of the species of Figures 5 through 14. For purposes of ready comparison, similar reference characters have been used for corresponding elements. The only difference between the arrangement of Figure 15 and that of Figures 5 through 14 is that instead of there being a tubular projection 56, there is a nipple type of projection 75. This nipple type of projection has an aperture 76 through it. The purpose of the aperture 76 is to provide a means for admitting air between the inner and outer members as the outer member 51 is withdrawn. Otherwise, there would be a tendency for a suction to be created. In the arrangement shown in Figures 5 through 13, this is provided for because the projection 56 is tubular and the air can be freely admitted between the inner and outer members. Where, however, a nipple 75 is employed for grasping the outer member 51 when it is desired to remove it, it is desirable to have a passage such as opening 76 to admit the air. While the device can function without such a passage, it does facilitate the removal of the outer member 51.

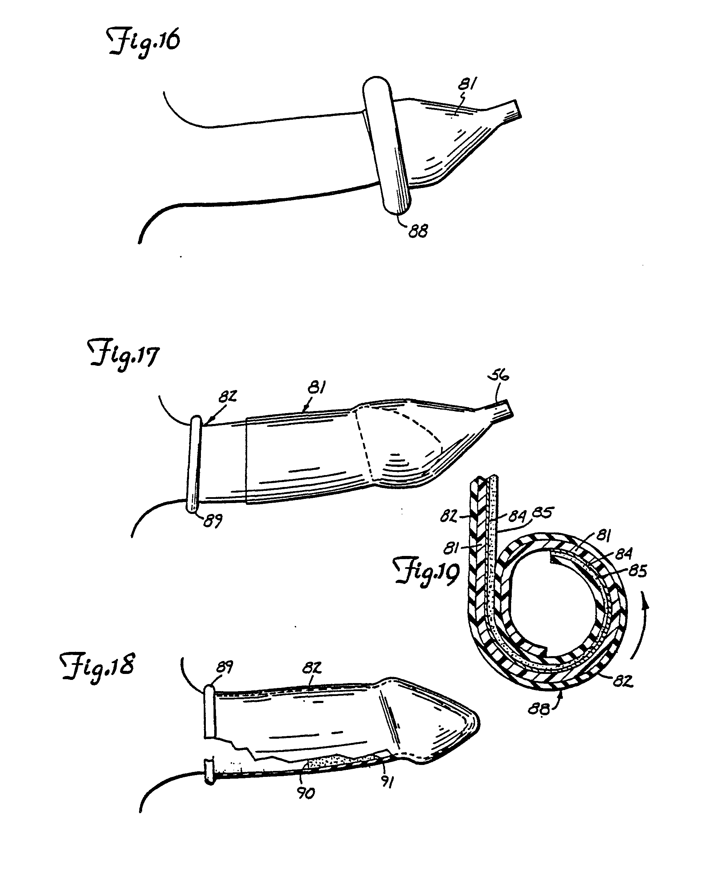

MODIFICATION OF FIGURES 16-18

The modification of Figures 16-18 is the same as that of Figures 5-15, except for the fact that the contraceptive hood is longer to extend over the full length of the penile shaft. While the outer member is longer than that of Figures 5-15, it is shorter than the inner member so that the outer member will be fully unrolled before the inner member reaches the base of the penile shaft.

Referring to Figures 16 and 17, the outer members is indicated by the reference numeral 81 and the inner member is indicated by the reference numeral 82. Initially, as shown in Figure 19, the outer member has a layer of adhesive 84 with a release layer 85 between the adhesive and the outer surface of the outer member 81. As best shown in Figure 17, in which the members have been unrolled, the outer member 81 in this modification is substantially shorter than the inner member 82. As in the previous species, it is thicker than the inner member. The relative thicknesses of the inner and outer members are substantially the same as in the species of Figures 5-15. The outer member, similar to member 51, has a conical outer portion 85 and an extending tubular portion 56 which acts as a means for withdrawal of the outer member 81 when it is the unrolled condition shown in Figure 7. Initially, the inner and outer members are rolled together, as in the preceding species, to form a roll 88. In Figure 19, there is a sectional view of the roll 88 with the members being shown in the process of being rolled up. It will be noted that the inner end of the outer member 81 does not extend as far into the roll as the outer member 82.

In use, the contraceptive hood is applied to the tip of the penis in the same manner as in the other species. This is shown in Figure 16. The contraceptive hood is then unrolled. Initially, the outer member 81 will be completely unrolled. The user then continues to unroll the inner member 82, normally leaving a small roll 89 at the base of the penis.

In the process of rolling and unrolling, the adhesive 85 is transferred from the outer surface of the outer member 81 to the inner surface of member 82 just as in the species of Figures 5-14. The primary difference, however, is that the layer of adhesive 90, shown in the broken away portion of Figure 18, extends much further than the layer of adhesive in the species of Figures 5-14, and as shown in Figure 14. In one particular instance, an adhesive layer of one and one-half inches was used. This started just back of the tip of the penis. In a typical case, the outer member 81 was about four inches long.

It will be obvious that the operation, except for the difference in length of the inner and outer members, is basically the same as in the species of Figures 5-14. When the unit has been unrolled to the position shown in Figure 17, the adhesive originally on the outer surface of member 81 has been transferred to the inner surface of the inner member 82. All that remains on the outer member is some of the adhesive release material. This is drawn off with the outer member. Thus, the outside of the inner sheath 81 is free of any release material or adhesive.

Because of the presence of the adhesive 90 over a substantial portion of the sheath, the sheath is held in place even after ejaculation has occurred. In this way, the contraceptive hood of this invention is much safer to use than the conventional type of hood which tends to become loose on the penis after ejaculation has occurred.

CONCLUSION

In conclusion, it will be seen that we have provided a contraceptive hood which is free of adhesive on the inner surface while the hood is first being applied to a penis. As the hood is unrolled, however, adhesive on an outer surface of a cylindrical member is transferred to an inner surface to cause the hood to firmly adhere to the penis adjacent the head of it.

While we have shown certain embodiments of the invention for purposes of illustration, it is to be understood that our invention is limited solely by the scope of the appended claims.

## Claims

1. A contraceptive hood to be adhesively applied to a penis, said contraceptive hood including:

a flexible cylindrical member (12) having a closed end and an open end;

said cylindrical member (12) being rolled outwardly upon itself from said open end to form consecutively larger rolls (13); and

an adhesive layer (15) and an adhesive release layer (14) disposed between the rolls (13) and the adhesive release layer (14) disposed between said adhesive layer (15) and the outer surface of said cylindrical member (12) in non-adhering contact with the adhesive layer (15) so that upon said member (12) being unrolled onto the head of a penis (20), the adhesive layer (15) adheres to the inner surface of said cylindrical member (12) to cause the cylindrical member (12) to adhere to the penis (20).

2. A contraceptive hood as claimed in claim 1 in which the cylindrical member comprises an inner thin cylindrical portion (52) and an outer relatively thick cylindrical portion (51) which bears the adhesive layer (15) and the release layer (14).

3. A contraceptive hood as claimed in claim 1 or 2 in which the adhesive release layer (14) is formed of a silicon compound.

4. A contraceptive hood as claimed in any of claims 1 to 3 in which the layer of adhesive (15) extends over only that portion adjacent to the open end which is engaged by the inner surface of the cylindrical member (12) when said cylindrical member (12) is in rolled up condition.

## Patentansprüche

1. Empfängnisverhütende Kappe, die haftend auf einem Penis angebracht wird, enthaltend

ein flexibles, zylindrisches Bauteil (12), das ein geschlossenes und ein offenes Ende hat;

wobei das zylindrische Bauteil (12) vom offenen Ende nach außen hin auf sich selbst unter Bildung fortlaufend größerer Rollen (13) aufgerollt wird; und

eine Haftschicht (15) und eine Haftfreigabeschicht (14), die zwischen den Rollen (13) angeordnet sind, wobei die Haftfreigabeschicht (14) zwischen der Haftschicht (15) und der äußeren Oberfläche des zylindrischen Bauteils (12) in nichthaftendem Kontakt mit der Haftschicht (15) angeordnet ist, so daß beim Entrollen des Bauteils (12) auf den Kopf eines Penis' (20) die Haftschicht (15) an der inneren Oberfläche des zylindrischen Bauteils (12) haften bleibt, was die Haftung des zylindrischen Bauteil (12) an dem Penis (20) bewirkt.

2. Empfängnisverhütende Kappe nach Anspruch 1, wobei das zylindrische Bauteil ein inneres, dünnes, zylindrisches Teilstück (52) und ein äußeres, verhältnismäßig dickes zylindrisches Teilstück (51), das die Haftschicht (15) und die Freigabeschicht (14) trägt, aufweist.

3. Empfängnisverhütende Kappe nach Anspruch 1 oder 2, wobei die Haftfreigabeschicht (14) aus einem Silicon besteht.

4. Empfängnisverhütende Kappe nach einem der Ansprüche 1 bis 3, wobei die Haftschicht (15) nur über das Teilstück reicht, welches an das offene Ende angrenzt und durch die innere Oberfläche des zylindrischen Bauteils (12) belegt ist, wenn das zylindrische Bauteil (12) in aufgerolltem Zustand ist.

## Revendications

1. Préservatif destiné à être appliqué par adhérence sur un pénis, le préservatif comprenant:

un organe cylindrique souple (12) ayant une extrémité fermée et une extrémité ouverte

l'organe cylindrique (12) étant enroulé vers l'extérieur sur lui-même depuis l'extrémité ouverte afin qu'il forme des enroulements de plus en plus gros (13), et

un couche adhésive (15) et une couche de séparation (14) disposées entre les enroulements (13), la couche (14) de séparation étant placée entre la couche adhésive (15) et la surface externe de l'organe cylindrique (12) sans adhérer à la couche adhésive (15), si bien que, lorsque l'organe (12) est déroulé sur l'extrémité d'un pénis (20), la couche adhésive (15) adhère à la surface interne de l'organe cylindrique (12) et provoque l'adhérence de l'organe cylindrique (12) au pénis (20).

2. Préservatif selon la revendication 1, dans lequel l'organe cylindrique a une mince partie cylindrique interne (52) et une partie cylindrique externe relativement épaisse (51) qui porte la couche adhésive (15) et la couche de séparation (14).

3. Préservatif selon la revendication 1 ou 2, dans lequel la couche de séparation (14) est formée d'un composé à base de silicone.

4. Préservatif selon l'une quelconque des revendications 1 à 3, dans lequel la couche adhésive (15) est disposée uniquement sur la partie adjacente à l'extrémité ouverte qui est au contact de la surface interne de l'organe cylindrique (12) lorsque l'organe cylindrique (12) est enroulé.

Fig.1

Fig.2

Fig.3

Fig.4

Fig.5

Fig.6

**Fig.7**

**Fig.8**

**Fig.9**

Fig.11

Fig.15

Fig.12

Fig.13

Fig.14

Fig.16

Fig.17

Fig.18

Fig.19

5